# EUROPÄISCHE PATENTSCHRIFT

(11) **EP 1 283 822 B1**
(45) Veröffentlichungstag und Bekanntmachung des Hinweises auf die Patenterteilung: **15.01.2014**
(21) Anmeldenummer: 01943291.3
(22) Anmeldetag: 26.04.2001
(51) Int. Cl.: C07C 39/235, C07C 39/44, C07C 37/88, A01N 31/08, A01N 31/16

(54) **PHENOLATE ENTHALTENDE FORMULIERUNG MIT TIEFEM GEFRIERPUNKT**
PHENOLATE-CONTAINING FORMULATION WITH LOW FREEZING POINT
FORMULATION CONTENANT DES PHENOLATES, AYANT UN POINT DE CONGELATION BAS

(30) Priorität: 12.05.2000 DE 10023458; 02.06.2000 DE 10027588
(43) Veröffentlichungstag der Anmeldung: 19.02.2003
(73) Patentinhaber: Omya International AG, 4665 Oftringen (CH)
(72) Erfinder: BURI, Matthias, 4852 Rothrist (CH); SCHWARZENTRUBER, Patrick, 4656 Starrkirch-Wil (CH)
(74) Vertreter: Richebourg, Michel François
(86) Internationale Anmeldenummer: PCT/EP2001/004729
(87) Internationale Veröffentlichungsnummer: WO 2001/085659

(56) Entgegenhaltungen:
- DE-A- 4 202 051
- GB-A- 804 257

## Beschreibung

Die Erfindung betrifft eine wässrige, Phenolate enthaltende flüssige Formulierung mit einem Erstarrungspunkt von kleiner oder gleich minus 10°C, ein Verfahren zu ihrer Herstellung, eine die Formulierung enthaltende wässrige Suspension oder Dispersion sowie verschiedene industrielle Verwendungen der Formulierung.

Phenolderivat-Formulierungen haben Mängel, speziell beim Einsatz als Konservierungsmittel im technischen Bereich, wie in hochkonzentrierten Pigmentslurries. Salze von Phenol, alkyl- und arylsubstituierten Phenolen, halogenierten Phenolen sowie Kresolen und halogenierten Kresolen sind bekannt als Fungizide und Bakterizide im protektiven und kurativen Bereich. Die meisten Formulierungen dieser Phenolate sind als wasserlösliche Alkalisalz-Formulierungen zu alkalisch für die spätere Anwendung und beeinflussen dadurch das zu schützende Produkt negativ oder haben einen Gefrierpunkt von 0°C oder nur wenig unter 0°C oder kristallisieren bei tiefer Temperatur und hohen Konzentrationen speziell bei Beimpfung mit Impfkristallen aus oder enthalten grosse Mengen an organischen Lösungsmittel oder hohe Mengen an Alkafilaugenüberschuss.

Speziell Natrium-o-phenylphenolat sowie Kalium-o-phenylphenolat sind bekannt als Fungizide für Holz, aber auch als Konservierungsmittel für Pigmentslurries. Natrium-o-phenylphenolat und Kalium-o-phenylphenolat sind in Pulverform erhältlich. Natrium-o-phenylphenolat ist weiterhin als 25 Gew.-%ige Natronlaugenlösung und als 35 - 38 Gew.-%ige Emulsion, unter Verwendung hoher Mengen an Emulgatoren zu deren Stabilisierung, auf dem Markt. Kalium-o-phenylphenolat ist als eine 35 - 39 Gew.%ige *Kalilaugenlösung* mit 38 Gew.% Kalium-o-phenylphenolat und 6 - 10 Gew.% Kalilauge in Wasser auf dem Markt.

In der DE 198 59 136.5 werden neuerdings auch teilneutralisierte Formen von o-Phenylphenol / Alkali-o-phenylphenolat in Wasser und grossen Mengen organischer Lösemittel, wie Glykolen und aromatischen Alkoholen, beschrieben. In der DE 42 022 051 A1 werden ebenfalls hochkonzentrierte flüssige Formen aus der Reihe der Phenolderivate beschrieben. Diese liegen jedoch in der nicht neutralisierten Form vor, besitzen einen Schmelzpunkt von über + 15 °C und sind nicht wasserlöslich.

Natrium-o-phenylphenolat in Pulverform ist in grösseren Mengen nicht leicht zu handhaben. Flüssige Formulierungen werden klar vorgezogen.

25 Gew.-% wässriges Natrium-o-phenylphenolat verursacht hohe Transport- und Lagerkosten. Höhere Konzentrationen können wegen zu geringer Löslichkeit nicht erreicht werden. Auch bei nur 25 % Konzentration neigt das Produkt bereits zu Auskristallisation bei Raumtemperatur. Bei Minustemperaturen kristallisiert es spontan aus. 35 - 39 Gew.-% Kalium-o-phenylphenolat mit einem hohen Überschuss an Kalilauge ist sehr ätzend und hat einen pH-Wert von weit über 12. Um eine Kristallisation bei minus 10°C zu verhindern, ist ein bis zu 30% iger Überschuss an Kalilauge notwendig. Dieser hohe pH-Wert und die hohe Ionenkonzentration führen bei der Zugabe zu einem wässrigen Pigmentslurry mit hohem Feststoffgehalt, speziell bei einer Konzentration von > 50 Volumen-% an Feststoff, zur Agglomeratbildung in der Pigmentslurry und zu einer Veränderung des pH Werts im Endprodukt.

Die in der DE 198 59 136.5 beschriebenen teilneutralisierten Phenolate, gelöst in Wasser und Glykolen, neigen bei ungenügender Scherung während des Einrührens zur Ausbildung von Agglomeraten, Trennung der wässerigen Pigmentslurry von der teilneutralisierten Phenolatlösung und zum Aufschwimmen derselben auf der Oberfläche der Slurry. Die Konservierung ist dadurch nicht optimal, und es kann zu Ablagerungen von Phenolaten in Rohrleitungen kommen. Der hohe Anteil an organischen Lösemitteln im Bereich von 20 - 90 Gewichts-% ist zudem bei manchen Anwendungen nicht erwünscht.

Die Verwendung von emulgiertem Natrium-o-phenylphenolat in Pigmentslurries mit hohem Feststoffgehalt ist riskant, da der Emulgator die Pigmentdispergierung destabilisiert und zu Schaumbildung neigt. Weiterhin liegt der Gefrierpunkt der meisten wässrigen Salzlösungen und wässrigen Emulsionen von o-Phenylphenol bei, oder nicht viel unterhalb 0°C. Nur Kalium-o-phenylphenolat mit einem starken Überschuss von KOH hat einen Gefrierpunkt von minus 15°C. Diese Verbindung weist jedoch das Risiko der Veränderung der Pigmentslurry - Eigenschaften, wie Agglomeratbildung und pH Erhöhung auf. Während des Winters in Nordeuropa, z.B. in Norwegen, und in Nordamerika und Kanada ist es unmöglich, wässrige Flossigkeiten mit einem Erstarrungspunkt um den Gefrierpunkt zu transportieren ohne zu riskieren, dass diese gefrieren, wenn keine Heizung am Transportbehälter installiert ist. Das gleiche Problem besteht bei der Lagerung. Weiterhin ist es ökonomisch und ökologisch unvernünftig und von der industrie nicht akzeptiert, derart niedrig konzentrierte Lösungen über weite Distanzen zu transportieren.

GB 804 257 beschreibt Phenolate und Kristallisationshemmer. Die maximale Löslichkeit von Na Pentachlorophenolat in 10% igem wäßrigem Ethylalkohol aber nur 27.3 Gew.-% beträgt.

Es ist eine Aufgabe der Erfindung, eine flüssige Formulierung von Phenolaten bereitzustellen, die einen Gefrierpunkt bzw. einen Erstarrungspunkt von kleiner oder gleich -10°C aufweist und deren Lösemittelsysem zu einem überwiegenden Anteil Wasser enthält.

Diese Aufgabe wird erfindungsgemäß durch die im Anspruch 1 näher gekennzeichnete flüssige, Phenolate enthaltende wässrige Formulierung gelöst, die einen Erstarrungspunkt von kleiner oder gleich -10°C aufweist. Die hier beschriebene Formulierung weist die nachfolgenden Bestandteile auf:
a) 50-80 Gew.-% eines oder mehrerer Phenolate;
b) 0,1-10 Gew.-% mindestens eines Kristallisationshemmers ausgewählt aus einer oder mehreren aliphatischen Glykolverbindungen wie Ethylenglycol, Monopropylenglykol und/oder Diethylenglycol, und/oder Benzylalkohol, 2-Phenylethan-1-ol, 3-Phenylpropan-1-ol und/oder 1-Phenylpropan-2-ol ; und die Differenz zu jeweils 100 Gew.-% der Alkaliüberschuss von 0.03-0.15 Mol/Mol und Wasser ist.

Bevorzugte Ausführungsformen der Erfindung ergeben sich aus den Unteransprüchen, den nebengeordneten Ansprüchen sowie aus der nachfolgenden Beschreibung.

Die erfindungsgemäße Formulierung zeichnet sich dadurch aus, daß sie Phenolate in konzentrierter Form von 50 - 80 Gew.-%, bezogen auf die gesamte Formulierung, enthält. Unter Phenolate werden Salze der Phenole verstanden, die sich in Wasser mit alkalischer Reaktion auflösen. Phenole ist die Gruppenbezeichnung für aromatische Hydroxyverbindungen, bei denen die Hydroxygruppen direkt an den Benzolkern gebunden sind. Beispiele für Phenole sind das Phenol selbst und einfach oder mehrfach substituierte Phenole mit aliphatischen und/oder aromatischen Substituenten. Beispiele hierfür wiederum sind o-Phenylphenol; Kresole und Resorcine. In der vorliegenden Formulierung liegen die Phenole und ihre Derivate in vollständig neutralisierter Form als Salze, d.h. als Phenolate, vor.

Weiterhin liegt bei der erfindungsgemäßen Formulierung der Phenolat-Gehalt, gerechnet als entsprechendes Phenol resp. Phenolderivat bei vorzugsweise über 40 Gew.-% . Die Phenolatlösung, vorzugsweise o-Phenylphenolat, ist in Bezug auf das Phenolat bevorzugt zu 103 - 115 Mol% neutralisiert, bevorzugt mit Alkalihydroxiden. Dies bedeutet, daß der Phenolatlösung pro Mol Phenolat 1,03 - 1,15 Mol alkalische Substanz, bevorzugt Alkalihydroxid zugesetzt wird. Insbesondere bevorzugt werden zur Neutralisation 105 Mol% KOH, bezogen auf den Phenolatgehalt, eingesetzt.

Die erfindungsgemäß eingesetzten Phenolate entfalten eine mikrobizide Wirkung und wirken somit als Konservierungsmittel. Die erfindungsgemäßen Formulierungen können aufgrund dieser Eigenschaften sowohl für den protektiven als auch für den kurativen Einsatz dienen.

Bevorzugte Phenolate sind einfach oder mehrfach substituierte Phenolate mit aliphatischen und/oder aromatischen Substituenten. Beispiele für derartige Derivate, die erfindungsgemäß einsetzbar sind, sind o-Phenylphenolat, halogenierte Phenolate, Salze von Kresolen, Salze von halogenierten Kresolen und Salze von Resorcinen oder deren Mischungen. Beispiele für Salze der Kresole sind Salze von halogenierten Kresolen, insbesondere Salze von chlorierten Kresolen, Salze von o-, m- und p-Kresol, Salze von Isopropyl-o-kresol, Salze von 4-Isopropyl-m-kresol. Ein Beispiel für ein verwendbares Salz der Resorcine ist ein Salz des 4-n-Hexylresorcin.

Die Phenolate liegen in einer Menge von 50-80 Gew.-% vor, wobei selbstverständlich alle Bereiche zwischen 50 und 80 Gew.-% von der Erfindung mit umfaßt werden. Bevorzugte Bereiche sind 50-75 Gew.-% 55 - 75 Gew.-%, 55 - 70 Gew.-% , 60 - 70 Gew.-%, 60 - 65 Gew.-%, 62-67 Gew.-% und besonders bevorzugt 65 Gew.-%, jeweils bezogen auf die gesamte Formulierung.

In einer bevorzugten Ausführungsform der Erfindung ist die Phenolatlösung mit Alkalihydroxiden neutralisiert worden, so daß die Phenolate bevorzugt als Kaliumsalz, Kalium- und Natriumsalz und/oder Kalium- und Lithiumsalz vorliegen. Insbesondere bevorzugt liegen die Phenolate als Kaliumsalz vor.

Das Lösungsmittelsystem für die Phenolate enthält bevorzugt einen Überschuß von 0,03 - 0,15 Mol an Alkalihydroxiden. Pro Mol Phenolat werden zur Neutralisation bevorzugt 1,03 - 1,15, weiterhin bevorzugt 1,05 - 1,10 Mol an Alkalihydroxiden eingesetzt. Der Neutralisationsgrad mit Alkalihydroxid, bevorzugt mit Kaliumhydroxid, bezogen auf das Phenolat, beträgt 102 - 115 Mol%, bevorzugt 103 - 107 Mol%, besonders bevorzugt 105 Mol%.

Entscheidend für den Erfolg der vorliegenden Erfindung ist die überraschende und nicht voraussehbare Wirkung geringer Mengen von Alkoholen, welche unerwartet als Kristallisationshemmer wirken.

Als Kristallisationshemmer werden organische wasserlösliche Substanzen, vorzugweise Alkohole, in Mengen von 0,1 - 10 %, oder Mischungen derselben, z.B. in Mengen von 1 bis 5 Gew.-% eingesetzt.

Als Kristallisationshemmer werden bevorzugt ein oder mehrere aliphatische Glykolverbindungen wie Ethylenglykol, Monopropylenglykol und/oder Diethylenglykol, und/oder ein oder mehrere aliphatische Alkohole wie Methanol, Ethanol, n-, isoPropanol, Isomere des Butanols, wie 1-Butanol und/oder des Pentanols und/oder ein oder mehrere aromatische Alkohole wie Benzylalkohol, 2-Phenylethan-1-ol, 3-Phenylpropan-1-ol und/oder 1-Phenylpropan-2-ol, eingesetzt.

Die Kristallisationshemmer liegen, bezogen auf die Gesamtformulierung, in einer Menge von 0,1 - 10 Gew.-% vor. Bevorzugte Mengen sind 0,5 - 5,0 Gew.-%, weiterhin bevorzugt 1 - 3 Gew.-%, bezogen auf die Gesamtformulierung, wobei selbstverständlich auch alle Bereiche zwischen 1 und 10 Gew.-% einsetzbar sind.

Beispiele für bevorzugt einsetzbare aromatische Alkohole sind:
Benzylalkohol und/oder 2-Phenylethan-1-ol und/oder 3-Phenylpropan-1-ol und/oder 1-Phenylpropan.
Beispiele für bevorzugt einsetzbare einwertige aliphatische Alkohole sind:
Methanol, Ethanol, Propanole, Butanole, Pentanole
Beispiele für bevorzugt einsetzbare aliphatische Glykole sind:
Ethylenglykol, Propylenglykol, Butandiole, Pentandiole.

Der Phenolatgehalt der erfindungsgemäßen Formulierung liegt, gerechnet als entsprechendes Phenol resp. Phenolderivat, bei vorzugsweise über 40 Gew.%. Die Phenolatlösungen, bei denen o-Phenylphenolat besonders bevorzugt wird, sind in Bezug auf das Phenolat bevorzugt zu 103 - 115 Mol% neutralisiert, bevorzugt mit Alkalihydroxiden, insbesondere mit KOH. In einer bevorzugten Ausführungsform werden zur Neutralisation 105 Mol% KOH, bezogen auf den Phenolatgehalt, eingesetzt.

Die erfindungsgemäße Formulierung enthält neben den Phenolaten und den Kristallisationshemmem, die einen Anteil von 50,1 - 90 Gew.-% ausmachen, 10 - 49,9 Gew.-% Wasser sowie wahlweise weitere Bestandteile wie weitere mikrobiozid wirkende Mittel und/oder mikrobiozid wirkende Mittel unterstützende Substanzen.

Bei der erfindungsgemäßen Formulierung handelt es sich um eine wässrige Formulierung, wobei das Lösungsmittelsystem, welches einen Anteil an der Formulierung von 20 - 50 Gew.-% hat, zu 90 - 99 Gew.-% aus Wasser besteht. Die Kristallisationshemmer sind Teil des Lösungsmittelsystems und liegen in einem Anteil von 0,1 - 10 Gew.-%, bezogen auf das Lösungsmittelsystem, vor. Es ist aber auch möglich, innerhalb dieser Grenzen einen Teil des Wassers bzw. des Kristallisationshemmers durch andere Bestandteile zu ersetzen, beispielsweise durch weitere mikrobiozide Mittel und mikrobiozide Mittel unterstützende Substanzen. Besonders bevorzugt werden als mikrobiozid wirkende Mittel eingesetzt: Amine, primäre und/oder sekundäre und/oder tertiäre und/oder quaternäre Amine und/oder Diamine, bevorzugt primäre und/oder sekundäre und/oder tertiäre und/oder quaternäre Fettamine und/oder Diamine, wobei ein oder mehrere Substituenten am Stickstoff eine Kettenlänge von 10 bis 20 Kohlenstoffatome, bevorzugt 10 bis 18 Kohlenstoffatome, aufweisen. Beispiele hierfür sind Dodekylamin, Didodekylamin, Didodekylmethylamin, Didodekylbenzylmethylammoniumchlorid, oder die Substanzen Dicocomethylbenzylammoniumchlorid, N -Tallow-1,3-diaminopropan. Die primären und/oder sekundären und/oder tertiären Fettamine und/oder die Amine können auch als Salze vorliegen. Als Neutralisationsmittel für die primären und/oder sekundären und/oder tertiären Amine und/oder die Diamine können Mineralsäuren und/oder organische Säuren eingesetzt werden, wobei bevorzugt Ameisensäure und/oder Essigsäure eingesetzt wird. Ein weiteres Beispiel für ein mikrobiozides Mittel ist Tributylzinnbenzoat. Beispiele für mikrobiozide Mittel unterstützende Substanzen wie Komplexbildner, bevorzugt Nitrilotriessigsäure, Ethylendiamintetraessigsäure, Diethylentriaminpentaessigsäure und deren Alkalisalze und wahlweise eine oder mehre Oxidationsstabilisatoren wie 2-Phosphono-1,2,4-butantricarbonsäure, bevorzugt in Mengen von 0,05 - 1,0 Gew.-%.

In einer 60 - 70 Gew.-% Phenolat enthaltenden Formulierung liegen die Kristallisationshemmer bevorzugt in einer Menge von 1 - 3 Gew.-%, bezogen auf die gesamte Formulierung, vor.

Die Phenolate in der Formulierung weisen eine mikrobiozide Wirkung auf und wirken somit als Konservierungsmittel. Aufgrund dieser Eigenschaften können sie sowohl für den protektiven als auch für den kurativen Einsatz dienen. Die Formulierung hat den Vorteil, daß die Phenolate nicht spontan oder allmählich auskristallisieren oder zumindest derart langsam, daß dies nicht nachteilig wirkt.

Die erfindungsgemäßen Formulierungen weisen einen Kristallisationspunkt resp. Gefrierpunkt, auch bei Zusatz von Kristallisationskeimen, von mindestens -10°C auf. Bevorzugte Ausgestaltungen der Formulierungen weisen Kristallisationspunkte resp. Gefrierpunkte von -15°C oder sogar -20°C auf.

Die Formulierung ist somit auch dadurch charakterisiert, daß sie
a) 50 - 80 Gew.-% zumindest eines Phenolats und
b) 20 - 50 Gew.-% eines Lösungsmittelsystems enthält, welches zu 90 - 99,9 Gew.-% aus Wasser und zu 0,1 - 10,0 Gew.-% aus zumindest einem Kristallisationshemmer besteht, wobei ein Anteil von 1,0 - 4,9 Gew.-% Gew.-% dieser Formulierung durch weitere mikrobiozide Mittel und/oder weitere Bestandteile ersetzt sein kann.

Entscheidend für den Erfolg der vorliegenden Erfindung war somit die überraschende und nicht voraussehbare Wirkung geringer Mengen von Alkoholen, die in einer Menge von 0,1 - 10 Gew.-%, bezogen auf die Gesamtformulierung, eingesetzt werden und die unerwartet in dieser Formulierung als Kristallisationshemmer wirken.

In weiteren Ausführungsformen der Erfindung sind in der Formulierung weitere mikrobizid wirkende Stoffe enthalten, beispielsweise bakterizid und/oder fungizid wirkende Verbindungen.

Bevorzugt enthält die erfindungsgemäße Formulierung keine Emulgatoren, anionaktive, kationaktive, nichtionogene Netzmittel, wie Laurylsulfat, Nonylphenole, Ethoxilate, Fettamine, da diese die zu konservierenden Suppensionen oder Dispersionen von Mineralien, Füllstoffen, Pigmenten und natürlichen oder synthetischen organischen Bindemitteln und deren Mischungen stören können, indem sie diese destabilisieren, eine Schaumbildung unterstützen und/oder zu Abscheidungen führen können.

Die erfindungsgemäßen Formulierungen enthalten als organische Lösungsmittelbestandteile neben dem Phenolat bevorzugt höchstens 1-10 Gew.-%, weiterhin bevorzugt 1-5 Gew.-%, ebenfalls bevorzugt 1-3 Gew.% organische Lösungsmittelbestandteile. Organische Lösungsmittelbestandteile bezieht sich hierbei auf den Kristallisationshemmer einschließlich der weiterhin wahlweise enthaltenen Bestandteile wie mikrobiozid wirkende Mittel und/oder mikrobiozid wirkende Mittel unterstützende Substanzen.

Die Herstellung der erfindungsgemäßen Formulierung kann vom Fachmann aufgrund seiner fachlichen Kenntnisse ohne erfinderische Schritte vorgenommen werden. Beispielsweise werden bei der Herstellung der Formulierung der vorliegenden Erfindung Wasser, das Neutralisationsmittel und der Kristallisationshemmer und eventuell weitere Substanzen in einem Gefäss vorgelegt und das Phenol unter Rühren und ev. Erwärmen gelöst:

Grundsätzlich ist die Reihenfolge der Zugabe gleich. Es kann jedoch zu einer vorübergehenden Unverträglichkeit kommen, die zu einer vorübergehenden Ausfällung von Stoffen führt. Bevorzugt werden deshalb das Wasser und das Neutralisationsmittel vorgelegt und die Phenolverbindungen darin gelöst und anschliessend der Kristallisationshemmer zugegeben.

Überraschend und unerwartet stellte sich heraus, dass mit Alkalihydroxiden vollständig neutralisierte, bevorzugt zu 103 - 115 Mol.% neutralisierte Phenolatlösungen und mit 0,1 -10 Gew.% Alkoholen als Kristallisationshemmer, bezogen auf die Gesamtformulierung, versehene Phenolatlösungen, bzw. von Salzen von Phenol und deren Derivate, z.B. , o-Phenylphenolat oder Salzen der Kresole, selbst bei hohem Feststoffgehalt von über 50 Gew.-% an Phenolaten, und sehr niedriger Temperatur von z.B. - 20°C auch durch mehrmaligen Zusatz von Keimbildnern, wie festem o-Phenylphenol nicht auskristallisierten, die Lösungen über Monate stabil blieben und sich eine geringere Braunfärbung bildete als sie zum Beispiel von herkömmlichen, wässerigen Lösungen von Alkalisalzen des o-Phenylphenols bestens bekannt ist und der Gefrierpunkt für die gestellte Aufgabe optimal ist. Im Gegensatz dazu sind die selben Formulierungen von Phenolaten überraschenderweise bei z.B. nur 40 Gew.-% an Phenolaten bei - 20°C nicht kristallisationsstabil sind und werden spontan fest.

Die erfindungsgemäße Formulierung wird, wie auch aus den anliegenden Beispielen ersichtlich ist, bevorzugt als Konservierungsmittel, ganz bevorzugt zur Konservierung wässriger Suspensionen oder Dispersionen von Mineralien, Füllstoffen, Pigmenten und natürlichen oder synthetischen organischen Bindemitteln und deren Mischungen eingesetzt. Suspensionen oder Dispersionen mit Feststoffgehalten von über 40 Gew.-%, bevorzugt über 60 Gew.-% und weiterhin bevorzugt über 70 Gew.-% können ohne Auskristallisieren der Phenolate mit der Formulierung der Erfindung auch bei Temperaturen von < - 10°C behandelt werden. Weiterhin ist sie zur Konservierung von Kühlschmierstoffen, bevorzugt in der Metall bearbeitenden Industrie, einsetzbar. Die die erfindungsgemäße Formulierung enthaltenden wässrigen Suspensionen oder Dispersionen von Mineralien, Füllstoffen und/oder Pigmenten werden bevorzugt in den Bereichen Papierherstellung, Papierbeschichtung und wässrige Lacke und Farben eingesetzt. Die Formulierung ist sowohl für den protektiven als auch für den kurativen Einsatz geeignet.

Die wässrige Suspension oder Dispersion kann weiterhin ein oder mehrere synthetische und/oder natürliche organische Bindemittel, bevorzugt Styrolbutadienlatices und/oder Styrolacrylatlatices, Stärke und/oder Carboxymethylcellulose, enthalten, die vor mikrobiellem Befall und/oder Verderb geschützt werden.

Die wässrige Suspension oder Dispersion enthält bevorzugt als Mineralien und/oder Füllstoffe und/oder Pigmente Elemente der zweiten und/oder dritten Hauptgruppe und/oder der vierten Hauptgruppe und/oder der vierten Nebengruppe des Periodensystems der Elemente, insbesondere Calcium und/oder Silicium und/oder Aluminium und/oder Titan und/oder Barium enthaltende Verbindungen und/oder organische Pigmente.

Die wässrige Suspension oder Dispersion enthält bevorzugt Kaolin und/oder Aluminiumhydroxid und/oder Titandioxid und/oder Bariumsulfat und/oder Polystyrolhohlkugeln und/oder Formaldehydharze und/oder Calciumcarbonat enthaltende Mineralien und/oder Füllstoffe und/oder Pigmente, insbesondere natürliche Calciumcarbonate und/oder präzipitierte Calciumcarbonate und/oder Marmor und/oder Kalk und/oder Dolomit und/oder Dolomit enthaltende Calciumcarbonate.

Die Erfindung betrifft somit auch wässrige Suspensionen oder Dispersionen von Mineralien und/oder Füllstoffen und/oder. Pigmenten und/oder natürlichen oder synthetischen organischen Bindemitteln und/oder Kühlschmierstoffen, die die Formulierung der vorliegenden Erfindung enthalten. Der Anteil der Formulierung in der wässrigen Suspension oder Dispersion beträgt bevorzugt 100 g Formulierung/Tonne zu konservierendes Gut bis 2500 g Formulierung/Tonne zu schützendes Gut.

Die Erfindung betrifft ebenfalls auch die Verwendung der erfindungsgemässen Formulierung als Konservierungsmittel in einer wässrigen Suspension oder Dispersion von Mineralien und/oder Füllstoffen und/oder Pigmenten und/oder natürlichen oder synthetischen organischen Bindemitteln und/oder Kühlschmierstoffen.

In einer bevorzugten Ausführungsform wird die erfindungsgemässe Formulierung als Konservierungsmittel in der Metall bearbeitenden Industrie, bei der Papierherstellung, Papierbeschichtung, in wässrigen Lacken und in Farben eingesetzt.

In einer weiteren bevorzugten Ausführungsform wird die erfindungsgemässe Formulierung als Konservierungsmittel und/oder Beizmittel in der Holz verarbeitenden Industrie und/oder im Forstbereich eingesetzt.

Ein weiterer Gegenstand der vorliegenden Erfindung betrifft das Verfahren zur Herstellung der erfindungsgemässen Formulierung, nämlich ein Verfahren zur Herstellung einer Phenolate enthaltende Formulierung in welcher das Wasser und das Neutralisationsmittel vorgelegt und die Phenolverbindungen darin gelöst werden und anschliessen der Kristallisationshemmer zugegeben wird.

Eine bevorzugte Ausführungsform des Verfahrens ist, dass der Kristallisationshemmer zusammen mit dem Wasser und dem Neutralisationsmittel vorgelegt werden, anschliessend die Phenolverbindungen darin gelöst werden.

Eine weitere bevorzugte Ausführungsform des Verfahrens ist, dass die Temperatur beim Lösungsvorgang 5-80°C, bevorzugt 40-60°C beträgt.

Nachfolgend wird die Erfindung anhand von Ausführungsbeispielen, auch im Vergleich mit dem Stand der Technik, weiter erläutert. Die Erfindung ist jedoch nicht auf diese beispielhaften Ausgestaltungen beschränkt.

### Allgemeine Bemerkungen zu den Beispielen

### 1.) Keimzählungen

Die Keimzahl wurde nach der Methode "Bestimmung von aeroben mesophilen Keimen", Schweizerisches Lebensmittelbuch, Kapitel 56, Abschnitt 7.01, Ausgabe 1985, Revision 1988, bestimmt. Die gefundenen Bakterienstämme stammen mehrheitlich aus der Familie der Pseudomonaden (vorwiegend Pseudomonas aeruginosa); aber auch gram-positive Keime und Pilze lagen vor.

### 2.) VISKOSITÄTSMESSUNG DER MINERAL- UND/ODER FÜLLSTOFF-UND/ODER PIGMENT-SUSPENSION:

Die Viskositätsmessung erfolgte auf einen Brookfield Viskosimeter Typ PVF-100 bei 100 U/Min. Für die einzelnen Messungen wurden die folgenden Spindeln verwendet:

| | |
|---|---|
| Spindel | RV 2 40 - 320 mPas |
| | RV 3 320 - 800 mPas |
| | RV 4 800 - 1600 mPas |
| | RV 5 1600 - 3200 mPas |
| | RV 6 3200 - 8000 mPas |

Die Messung erfolgte in einem 400 ml-Becherglas von niedriger Form.

Die Temperatur während der Messung betrug 20°C. Die Messung erfolgte nach 1 Min. Rührzeit.

Vor den eigentlichen Messungen wurden alle Proben 2 Min. intensiv gerührt (5000 U/Min., Rührscheibendurchmesser 50 mm).

Diese Art der Viskositätsmessung wurde für alle folgenden Beispiele verwendet.

### 3.) FEINHEIT DER MINERAL- UND/ODER FÜLLSTOFF- UND/ODER PIGMENT-SUSPENSION:

Die Feinheitsmerkmale der erfindungsgemäß hergestellten Suspensionen wurden durch Sedimentationsanalyse im Schwerefeld mit dem SEDIGRAPH 5100 der Firma Micromeritics, U.S.A., bestimmt.

Die Messung der kationisch stabilisierten Suspensionen erfolgte in destilliertem Wasser. Die Dispergierung der Proben wurde mittels Schnellrührer und Ultraschall vorgenommen.

Die Messung der Pulver erfolgte in 0,1% Na₄P₂O₇-Lösung

Die gemessene Teilchenverteilung wurde auf einem X-Y-Schreiber als Durchgangs-Summenkurve dargestellt (siehe z.B. Belger, P., Schweizerische Vereinigung der Lack- und Farben-Chemiker, XVII. FATIPEC-Kongreß, Lugano, 23. bis 28. September 1984), wobei auf der X-Achse der Teilchendurchmesser eines entsprechenden sphärischen Durchmessers und auf der Y-Achse der Anteil an Teilchen in Gew.-% aufgetragen wurde.

### 4.) HERSTELLUNG DER PHENOLATLÖSUNGEN

Die entsprechende Menge an demin. Wasser wurde vorgelegt und darin die berechnete Menge an Alkalilauge gelöst. Die Menge an Alkalilauge wurde so berechnet, dass das Phenol zu 105 Mol-% mit Kaliumhydroxid neutralisiert wird, ausser bei den Beispielen 2 und 3 zum Stand der Technik, wo 135 Mol% Kaliumhydroxid verwendet wurden.
Anschliessend wurde entsprechend des geforderten Aktivgehaltes der Lösung in (Gew.-%) das Phenol oder dessen Derivat zugefügt und unter Rühren und Erwärmen auf 50°C gelöst.
Bei den erfinderischen Beispielen wurden je nach Versuchreihe zwischen 1-10 Gew.-% Kristallisationshemmer zugegeben.
Beim Stand der Technik wurde kein Kristallisationshemmer zugesetzt.

Die Lösungen wurden anschliessend im Gefrierschrank während mindestens 24 Std. gelagert, visuell auf Kristallisation geprüft, mit 50 - 100 mg des entsprechenden trockenen Phenolats oder Phenolderivats als Keimbildner versetzt und bei - 20°C wieder auf Kristallisation geprüft.

### Beispiele zum Stand der Technik

### Beispiel 1, Stand der Technik

Probelösung 30 Gew.%ig OPP, entsprechend 39 Gew.% OPPK :

| | |
|---|---|
| 300,0 g | o-Phenylphenol |
| 103,5 g | KOH |
| 596,5 g | Wasser |

Probelösung 50 Gew.%ig OPP, entsprechend 66.5 Gew.% OPPK:

| | |
|---|---|
| 500,0 g | o-Phenylphenol |
| 173,0 g | KOH |
| 327,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst.

### Resultate :

| Probelösung | Kristallisation bei -20°C |
|---|---|
| 30 Gew.% o-Phenylphenol (OPP) (105 Mol-% K-neutr.) | ja |
| 50 Gew.% o-Phenylphenol (OPP) (105 Mol-% K-neutr.) | ja |

Eine 30, resp. 50 Gew.%ige Lösung von o-Phenylphenol, entsprechend 39 Gew.%, resp. 66,5 Gew.-% Kalium-o-phenylphenolat, neutralisiert mit 1,05 Mol KOH pro Mol o-Phenylphenol in destilliertem Wasser, kristallisiert teilweise spontan, teilweise nach Lagerung über 3 Tage bei - 20°C.
Bei Zugabe von 50 mg o-Phenylphenol (OPP Kristalle) als Keimbildner kristallisiert die Lösung spontan bei - 20°C zu einer festen Masse.
Die Lösung ist so nicht brauchbar.
Ohne grossen Überschuss an KOH ist die Lösung bei - 20°C nicht kristallisationsfrei.

### Beispiel 2, Stand der Technik

Probelösung 20 Gew.%ig OPP, entsprechend ca. 25 Gew.% OPPNa :

| | |
|---|---|
| 200,0 g | o-Phenylphenol |
| 49,4 g | NaOH |
| 750,6 g | Wasser |

Das Wasser wurde vorgelegt, die NaOH zugegeben und innert 5 Min. unter Rühren auf dem Magnetrührer gelöst. Anschliessend wurde das o-Phenylphenol zugegeben und bei 50°C unter Rühren auf dem Magnetrührer in der NaOH Lösung gelöst.

### Resultate :

| Probelösung | Kristallisation bei -20°C |
|---|---|
| 20 Gew.% o-Phenylphenol, (105 Mol-% Na-neutr.) | ja, spontan |
| entsprechend 24,7 Gew.% OPPNa | der Gefrierpunkt liegt bei - 7°C |

Eine 20 Gew.%ige Lösung von o-Phenylphenol, entsprechend 24,7 Gew.%iges Natrium-o-phenylphenolat, neutralisiert mit 1,05 Mol NaOH pro Mol o-Phenylphenol in destilliertem Wasser, kristallisiert spontan,der Gefrierpunkt liegt bei nur minus 7°C. Die Lösung ist so nicht brauchbar.

### Beispiel 3, Stand der Technik

Probelösung 30 Gew.%ig OPP, entsprechend 39 Gew.% OPPK:

| | |
|---|---|
| 300,0 g | o-Phenylphenol |
| 133,4 g | KOH entsprechend 1.35 Mol KOH / Mol OPP |
| 566,6 g | Wasser |

Das Wasser wurde vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst.

### Resultat :

| Probelösung | Kristallisation bei -20°C |
|---|---|
| Handelsübliche 30 Gew.%ige o-Phenylphenollösung, entsprechend einer 39 Gew.% Kalium-o-phenylphenolat-Lösung (1,35 Mol KOH/Mol OPP) | keine Kristallisation sichtbar |

Eine wässrige Aufschlämmung von Kaolin aus Georgia, USA, mit einem Feststoffgehalt von 72,8 Gew.-% und einer Korngrössenverteilung, so dass 94 Gew.-% der Partikel einen Durchmesser von weniger als 2 µm hatten (gemessen mit Sedigraph 5100, Micromeritics, USA), dispergiert mit 0,35 Gew.-% Natriumpolyacrylat und mit einem pH-Wert von 7.4 wurde mit 300 g, bezogen auf 100%-iges OPP der obigen handelsüblichen 39 gew.-%igen, resp. 66,5 gew.-%igen Kalium-o-phenylphenolat Lösung / t Slurry (entspricht 300 ppm 100%-iges OPP) versetzt. Eine Blindprobe der Kaolinslurry wurde auf die gleiche Art hergestellt, jedoch ohne Konservierungsmittel.

### Resultate:

| | pH | Viskosität Brookfield Spindel 3, 100 Upm nach der Produktion | Viskosität Brookfield Spindel 3, 100 Ppm nach 1 Woche |
|---|---|---|---|
| Blindprobe ohne | 7,4 | 240 mPas | 330 mPas |
| Kalium-o-phenylphenolat | | | |
| Probe mit 300 g aktiv OPP, 39 Gew.%ige Kalium-o-phenylphenolat / t Slurry | 8,1 | 310 mPas | 960 mPas |

In der Blindprobe ohne handelübliches Kalium-o-phenylphenolat wurde nach 48 Std. eine Keimzahl von 10⁵/g gemessen.
In der Probe mit 300 ppm, bezogen auf 100%-iges OPP, handelsüblichen Kalium-o-phenylphenolat wurde nach 48 Std. eine Keimzahl von < 100/g gemessen.

Die Viskosität des Kaolinslurry mit hohem Feststoffgehalt, dispergiert mit Natriumpolyacrylat, erhöhte sich bei Zugabe von 300 ppm, bezogen auf 100%-iges OPP der 39 gew.%igen, handelsüblichen Kalium-o-phenylphenolatlösung sofort und über eine Lagerzeit von 1 Woche.
In diesem Fall besteht die Gefahr, dass der Slurry nach einem mehrwöchigen Transport in grossen Mengen per Schiff, Bahn oder LKW nicht entladen werden kann. 300 ppm, bezogen auf 100%-iges OPP, zugegeben als Kalium-o-phenylphenolat, sind jedoch notwendig, um den Slurry keimfrei zu halten. Es ist nicht möglich, den Slurry zu konservieren, ohne die anderen Eigenschaften des Slurry negativ zu beeinflussen.

### Beispiel 4, Stand der Technik

Eine wässrige Aufschlämmung von Calciumcarbonat aus natürlichem Marmor aus Norwegen mit einem Feststoffgehalt von 77,8 Gew.-% und einer Korngrössenverteilung, so dass 90 Gew.-% der Teilchen einen Durchmesser von weniger als 2 µm hatten (gemessen mit Sedigraph 5100, Micromeritics, USA), wurde mit 250 g, bezogen auf 100%-iges OPP als handelsübliche, 39 Gew.%ige Kalium-o-phenylphenolatlösung wie in Beispiel 2 / t Slurry durch zutropfen unter Rühren innert 1 Min. konserviert. Eine Blindprobe der Calciumcarbonatslurry wurde auf die gleiche Weise hergestellt, jedoch ohne Konservierungsmittel.

### Resultate:

| | pH | Viskosität Brookfield Spindel 3, 100 Upm nach der Produktion | Viskosität Brookfield Spindel 3, 100 Upm nach 1 Woche |
|---|---|---|---|
| Blindprobe ohne | 9,6 | 340 mPas | 350 mPas |
| Kalium-o-phenylphenolat | | | |
| Probe mit 250 g | 10,4 | 320 mPas | 460 mPas |
| Kalium-o-phenylphenolat / t | | | |

Der Siebrückstand der Blindprobe ohne handelübliches Kalium-o-phenylphenolat betrug 25 ppm auf einem Sieb mit 45 µm Maschenweite.

Der Siebrückstand der Probe mit 250 ppm, bezogen auf 100%-iges OPP, zugegeben als handelübliches Kalium-o-phenylphenolat, betrug 160 ppm auf einem Sieb mit 45 µm Maschenweite.

In der Blindprobe ohne handelübliches Kalium-o-phenylphenolat wurde nach 48 Std. eine Keimzahl von 10⁵/g gemessen.

In der Probe mit 250 ppm, bezogen auf 100%-iges OPP, zugegeben als handelsübliches Kalium-o-phenylphenolat / t, wurde nach 48 Std. eine Keimzahl von < 100/g gemessen.

Die Viskosität des Calciumcarbonatslurry mit hohem Feststoffgehalt, dispergiert mit Natriumpolyacrylat, erhöhte sich nicht sehr stark über eine Lagerzeit von 1 Woche. Es war jedoch ersichtlich, dass der Siebrückstand auf dem 45 µm-Sieb unannehmbar anstieg. Die hohe Salzionenkonzentration führte zu Agglomeratbildung im hochkonzentrierten Slurry. Der pH-Wert des Slurry veränderte sich negativ in den alkalischen Bereich. Ein pH-Wert über 10 führt in der Streichfarbe der Papierindustrie zu Rheologieproblemen. Weiterhin führt der erhöhte Siebrückstand eines solches Produkts zwangsläufig zu Kratzern in der Papierbeschichtung und Stauben während dem Drucken.

Um den Slurry vor Verderb durch Mikroorganismen zu schützen, sind 250 ppm, bezogen auf 100%-iges OPP, zugegeben als handelsübliches Kalium-o-phenylphenolat aus Beispiel 2, notwendig.

Es ist nicht möglich, den Slurry zu konservieren, ohne andere Eigenschaften des Slurry negativ zu beeinflussen.

### Beispiel 5, Stand der Technik

Probelösung 30 Gew.%ig + 3 % Monopropylenglykol :

| | |
|---|---|
| 300,0 g | o-Phenylphenol |
| 103,5 g | KOH |
| 30,0 g | Monopropylenglykol |
| 596,5 g | Wasser |

Probelösung 30 Gew.%ig + 5 % Monopropylenglykol :

| | |
|---|---|
| 300,0 g | o-Phenylphenol |
| 103,5 g | KOH |
| 50,0 g | Monopropylenglykol |
| 546,5 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das Monopropylengykol zugegeben.

### Resultate :

| Probelösung | Kristallisation bei -20°C |
|---|---|
| | Unter Impfen mit 100 mg |
| | OPP als Impfkristalle |
| 30 Gew.% o-Phenylphenol (OPP) (105 Mol-% K-neutr.) | |
| mit 3 % Monopropylenglykol | spontane Kristallisation |
| mit 5 % Monopropylenglykol | spontane Kristallisation |

Eine 30 Gew.%ige Lösung von o-Phenylphenol, neutralisiert mit 1,05 Mol KOH pro Mol o-Phenylphenol, entsprechend 39 Gew.% Kalium-o-phenylphenolat, in destilliertem Wasser kristallisiert trotz Zugabe von 3 - 5 Gew.-% Monopropylenglykol durch OPP als Impfkristalle bei - 20°C spontan aus.

### Erfindungsgemässe Beispiele

### Beispiel 6,

Probelösung 50 Gew.%ig + 1 % Monopropylenglykol :

| | |
|---|---|
| 500,0 g | o-Phenylphenol |
| 173,0 g | KOH |
| 10,0 g | Monopropylenglykol |
| 317,0 g | Wasser |

Probelösung 50 Gew.%ig + 3 % Monopropylenglykol :

| | |
|---|---|
| 500,0 g | o-phenylphenol |
| 173,0 g | KOH |
| 30,0 g | Monopropylenglykol |
| 297,0 g | Wasser |

Probelösung 50 Gew.%ig + 5 % Monopropylenglykol :

| | |
|---|---|
| 500,0 g | o-Phenylphenol |
| 173,0 g | KOH |
| 50,0 g | Monopropylenglykol |
| 277,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das Monopropylengykol zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (OPP) (105 Mol-% K-neutr.) | |
| mit 1 % Monopropylenglykol | keine Kristallisation |
| mit 3 % Monopropylenglykol | keine Kristallisation |
| mit 5 % Monopropylenglykol | keine Kristallisation |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe von OPP als Impfkristalle bei - 20°C nicht aus, wenn der erfindungsgemässe Kristallisationshemmer in Form von Monopropylenglykol zugegeben wird.

### Beispiel 7, (Vergleichbeispiele)

Probelösung 50 Gew.%ig + 1 % 1-Butanol :

| | |
|---|---|
| 500,0 g | o-Phenylphenol |
| 173,0 g | KOH |
| 10,0 g | 1-Butanol |
| 317,0 g | Wasser |

Probelösung 50 Gew.%ig + 3 % 1-Butanol :

| | |
|---|---|
| 500,0 g | o-Phenylphenol |
| 173,0 g | KOH |
| 30,0 g | 1-Butanol |
| 297,0 g | Wasser |

Probelösung 50 Gew.%ig + 5 % 1-Butanol :

| | |
|---|---|
| 500,0 g | o-Phenylphenol |
| 173,0 g | KOH |
| 50,0 g | 1-Butanol |
| 277,0 g | Wasser |

Probelösung 50 Gew.%ig + 10 % 1-Butanol:

| | |
|---|---|
| 500,0 g | o-Phenylphenol |
| 173,0 g | KOH |
| 100,0 g | 1-Butanol |
| 227,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das 1-Butanol zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (OPP) (105 Mol-% K-neutr.) | |
| mit 1 % 1-Butanol | keine Kristallisation |
| mit 3 % 1-Butanol | keine Kristallisation |
| mit 5 % 1-Butanol | keine Kristallisation |
| mit 10 % 1-Butanol | keine Kristallisation |

Eine 50 Gew.%ige Lösung von o-Phenylphenol, neutralisiert mit 1,05 Mol KOH pro Mol o-Phenylphenol, entsprechend 66,5 Gew.% Kallum-o-phenylphenblat, in destilliertem Wasser kristallisiert auch unter Zugabe von OPP als Impfkristalle bei - 20°C nicht aus wenn der erfindungsgemässe Kristallisationshemmer in Form von 1-Butanol zugegeben wird.

### Beispiel 8,

Probelösung 50 Gew.%ig + 1 % Benzylalkohol:

| | |
|---|---|
| 500,0 g | o-Phenylphenol |
| 173,0 g | KOH |
| 10,0 g | Benzylalkohol |
| 317,0 g | Wasser |

Probelösung 50 Gew.%ig + 3 % Benzylalkohol:

| | |
|---|---|
| 500,0 g | o-Phenylphenol |
| 173,0 g | KOH |
| 30,0 g | Benzylalkohol |
| 297,0 g | Wasser |

Probelösung 50 Gew.%ig + 5 % Benzylalkohol:

| | |
|---|---|
| 500,0 g | o-Phenylphenol |
| 173,0 g | KOH |
| 50,0 g | Benzylalkohol |
| 277,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde der Benzylalkohol zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (OPP) (105 Mol-% K-neutr.) | |
| mit 1 % Benzylalkohol | keine Kristallisation |
| mit 3 % Benzylalkohol | keine Kristallisation |
| mit 5 % Benzylalkohol | keine Kristallisation |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe von OPP als Impfkristalle bei - 20°C nicht aus, wenn der erfindungsgemässe Kristallisationshemmer in Form von Benzylalkohol zugegeben wird.

### Beispiel 9 (Vergleichbeispiel)

Probelösung 50 Gew.%ig + 1 % 1-Butanol :

| | |
|---|---|
| 500,0 g | o-Phenylphenol |
| 173,0 g | KOH |
| 10,0 g | 1-Butanol |
| 317,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das 1-Butanol zugegeben.
Eine wässrige Aufschlämmung von Kaolin aus Georgia, USA, mit einem Feststoffgehalt von 72,8 Gew.-% und einer Korngrössenverteilung, so dass 94 Gew.-% der Partikel einen Durchmesser von weniger als 2 µm hatten (gemessen mit Sedigraph 5100, Micromeritics, USA), dispergiert mit 0,35 Gew.-% Natriumpolyacrylat und mit einem pH-Wert von 7,4 wurde mit 300 g, bezogen auf 100%-iges OPP, zugegeben als obige 66,5 gew.%ige Kalium-o-phenylphenolat Lösung / t Slurry, versetzt. Eine Blindprobe der Kaolinsluny wurde auf die gleiche Art hergestellt, jedoch ohne Konservierungsmittel.

### Resultate:

| | pH | Viskosität Brookfield Spindel 3, 100 Upm nach der Produktion | Viskosität Brookfield Spindel 3, 100 Ppm nach 1 Woche |
|---|---|---|---|
| Blindprobe ohne | 7,4 | 240 mPas | 330 mPas |
| Kalium-o-phenylphenolat | | | |
| Probe mit 300 g aktiv, der 66.5 Gew.%igen Kalium-o-phenylphenolat/ t Slurry | 7,6 | 265 mPas | 360 mPas |

In der Blindprobe ohne Kalium-o-phenylphenolat wurde nach 48 Std. eine Keimzahl von 10⁵/g gemessen.

In der Probe mit je 300 ppm, bezogen auf 100%-iges OPP, zugegeben als Kalium-o-phenylphenolat / t Slurry, wurde nach 48 Std. eine Keimzahl von < 100/g gemessen.

Die Viskosität des Kaolinslurry mit hohem Feststoffgehalt, dispergiert mit Natriumpolyacrylat, erhöhte sich bei Zugabe von jeweils 300 ppm, bezogen auf 100%-iges OPP, der erfindungsgemässen Kalium-o-phenylphenolat Lösung nur gering. In diesem Falle besteht keine Gefahr, dass der Slurry nach einem mehrwöchigen Transport in grossen Mengen per Schiff, Bahn oder LKW nicht entladen werden kann.

300 ppm, bezogen auf 100%-iges OPP der erfindungsgemässen Kalium-o-phenylphenolat sind ausreichend um den Slurry keimfrei zu halten. Es ist möglich, den Slurry zu konservieren, ohne andere Eigenschaften des Slurry negativ zu beeinflussen.

### Beispiele 10 (Vergleichsbeispiel)

Probelösung 50 Gew.%ig + 1 % 1-Butanol :

| | |
|---|---|
| 500,0 g | o-Phenylphenol |
| 173,0 g | KOH |
| 10,0 g | 1-Butanol |
| 317,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das 1-Butanol zugegeben.

Eine wässrige Aufschlämmung von Calciumcarbonat aus natürlichem Marmor aus Norwegen mit einem Feststoffgehalt von 77,8 Gew.-% und einer Korngrössenverteilung, so dass 90 Gew.-% der Teilchen einen Durchmesser von weniger als 2 µm hatten (gemessen mit Sedigraph 5100, Micromeritics, USA), wurde mit 250 g, bezogen auf 100%-iges OPP, der erfindungsgemässen 66,5 gew.%igen Kalium-o-phenylphenolat / t Slurry durch Zutropfen unter Rühren innert 1 Min. konserviert. Eine Blindprobe der Calciumcarbonatslurry wurde auf die gleiche Weise hergestellt, jedoch ohne Konservierungsmittel.

### Resultate:

| | pH | Viskosität Brookfield Spindel 3, 100 Upm nach der Produktion | Viskosität Brookfield Spindel 3, 100 Upm nach 1 Woche |
|---|---|---|---|
| Blindprobe ohne | 9,6 | 340 mPas | 350 mPas |
| Kalium-o-phenylphenolat | | | |
| Probe mit 250 g aktiv der 66.5 Gew.%ige Kalium-o-phenylphenolat / t Slurry | 9,8 | 290 mPas | 360 mPas |

Der Siebrückstand der Blindprobe ohne Kalium-o-phenylphenolat betrug 28 ppm auf einem Sieb mit 45 µm Maschenweite.

Der Siebrückstand der Probe mit 250 ppm, bezogen auf 100%-iges OPP, zugegeben als 66,5 gew.%ige erfindungsgemässe Kalium-o-phenylphenolatlösung, betrug 38 ppm auf einem Sieb mit 45 µm Maschenweite.

In der Blindprobe ohne Kalium-o-phenylphenolat wurde nach 48 Std. eine Keimzahl von 10⁶/g gemessen.

In der Probe mit 250 ppm, bezogen auf 100%-iges OPP, zugegeben als erfindungsgemässe Kalium-o-phenylphenolat / t Slurry, wurde nach 48 Std. eine Keimzahl von je < 100/g gemessen.

Die Viskosität des Calciumcarbonatslurry mit hohem Feststoffgehalt, dispergiert mit Natriumpolyacrylat, erhöhte sich nicht über eine Lagerzeit von 1 Woche. Sie lag innerhalb der Schwankungsbreite der Methode. Der Siebrückstand auf dem 45 µm-Sieb stieg nur sehr gering an und liegt ebenfalls in der Schwankungsbreite der Methode. Die geringere Salzionenkonzentration in der erfindungsgemässen Lösung führte zu keiner nennenswerten Agglomeratbildung im hochkonzentrierten Slurry. Der pH-Wert des Slurry veränderte sich nicht entscheidend in den alkalischen Bereich. Die Slurry, konserviert mit den erfindungsgemässen Formulierungen, ist für die Papierindustrie geeignet.

Um die Slurry vor Verderb durch Mikroorganismen zu schützen, sind 250 ppm, bezogen auf 100%-iges OPP, zugesetzt als Kalium-o-phenylphenolat, notwendig. Es ist mit der erfindungsgemässen Formulierung möglich, den Slurry zu konservieren, ohne andere Eigenschaften des Slurry negativ zu beeinflussen.

### Beispiel 11,

Probelösung 50 Gew.%ig, gerechnet als o-Phenylphenol + 3 % Monopropylenglykol :

| | |
|---|---|
| 500,0 g | o-Phenylphenol |
| 86,5 g | KOH |
| 60,3 g | NaOH |
| 30,0 g | Monopropylenglykol |
| 323,2 g | Wasser |

Das Wasser wurde jeweils vorgelegt, die KOH und NaOH zugegeben und innert 5 min unter Rühren auf dem Magnetrührer gelöst. Anschliessend wurde das o-Phenylphenol zugegeben und bei 50°C unter Rühren auf dem Magnetrührer in der KOH / NaOH Lösung gelöst. Zum Schluss wurde das Monopropylengykol zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (52,5 Mol% K-neutr.und 52,5 Mol-% Na-neutr.) mit 3 % Monopropylenglykol | keine Kristallisation |

Eine 50 Gew.%ige Lösung von o-Phenylphenol, neutralisiert mit je 0,525 Mol KOH respektive und 0,525 Mol NaOH pro Mol o-Phenylphenol, entsprechend ca. 64 Gew.% Kalium/Natrium-o-phenylphenolat, in destilliertem Wasser kristallisiert auch unter Zugabe von OPP als Impfkristalle bei - 20°C nicht aus, wenn der erfindungsgemässe Kristallisationshemmer in Form von Monopropylenglykol zugegeben wird.

### Beispiel 12, (Vergleichsbeispiel)

Probelösung 50 Gew.%ig, gerechnet als o-Phenylphenol + 3 % 1-butanoyl :

| | |
|---|---|
| 500,0 g | o-Phenylphenol |
| 173,0 g | KOH |
| 30,0 g | 1-Butanol |
| 297,0 g | Wasser |

Das Wasser wurde jeweils vorgelegt, die KOH zugegeben und innert 5 min unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das 1-Butanol zugegeben..

### Resultate :

| | |
|---|---|
| Art der Impfkristalle | Kristallisation bei -15°C |
| | Unter Impfen mit 100 mg verschiedener Phenole / Phenolate, Kresole, Resorcine oder anderer Substanzen als Impfkristalle |
| | |
| mit Phenol in Schuppenform | keine Kristallisation |
| mit o-Phenylphenol in Schuppenform | keine Kristallisation |
| mit Natrium-o-phenylphenolat in Schuppenform | keine Kristallisation |
| mit Kalium-o-phenylphenolat in Pulverform | keine Kristallisation |
| mit 4-Isopropyl-m-kresol in Pulverform | keine Kristallisation |
| mit 4-n-Hexylresorcin in Pulverform | keine Kristallisation |
| | |
| mit Quarzsand in Pulverform (50 Gew.-% < 2µm) | keine Kristallisation |
| mit o-Phenylphenol und Quarzsand in Pulverorm (50 Gew.-% < 2µm) | keine Kristallisation |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe verschiedener Arten von Impfkristallen bei minus 15°C nicht aus wenn der erfindungsgemässe Kristallisationshemmer in Form von 3 Gew.% 1-Butanol zugegeben wird.

### Beispiel 13,

Probelösung 40 Gew.%ig + jeweils 10 Gew.-% verschieden Kristallisationshemmer :

| | |
|---|---|
| 400,0 g | o-Phenylphenol |
| 138,4 g | KOH |
| 100,0 g | Monopropylenglykol, resp. 1-Butanol, resp. Benzylalkohol |
| 361,6 g | Wasser |

Das Wasser wurde jeweils vorgelegt, die KOH zugegeben und innert 5 Min. unte Rühren auf dem Magnetrührer gelöst. Anschliessend wurde das o-Phenylpheno zugegeben und bei 50°C unter Rühren auf dem Magnetrührer in der KOH Lösung gelöst. Zum Schluss wurde der Kritallisationshemmer zugegeben.

### Resultate :

| | |
|---|---|
| Probelösung | |
| | Kristallisation bei -10°C Unter Impfen mit 100 mg OPP als Impfkristalle |
| | |
| 40 Gew.% o-Phenylphenol (OPP) (105 Mol-% K-neutr.) | |
| mit 10 % Monopropylenglykol | keine Kristallisation |
| mit 10 % 1-Butanol | keine Kristallisation |
| mit 10 % Benzylalkohol | keine Kristallisation |

Eine 40 Gew.%ige Lösung von o-Phenylphenol, neutralisiert mit 1,05 Mol KOH pro Mol o-Phenylphenol, entsprechend 53 Gew.% Kalium-o-phenylphenolat, in destilliertem Wasser kristallisiert auch unter Zugabe von OPP als Impfkristalle bei -10°C nicht aus wenn die erfindungsgemässen Kristallisationshemmer in Form von Monopropylenglykol, resp. 1-Butanol, resp. Benzylalkohol zugegeben wird.

### Beispiel 14

Probelösung 50 Gew.%ig + 5 % Monopropylenglykol :

| | |
|---|---|
| 500,0 g | Phenol |
| 312,8 g | KOH |
| 50,0 g | Monopropylenglykol |
| 137,2 g | Wasser |

Das Wasser wurde jeweils vorgelegt, die KOH zugegeben und innert 5 Min. unter Rühren auf dem Magnetrührer gelöst. Anschliessend wurde das Phenol zugegeben und bei 50°C unter Rühren auf dem Magnetrührer in der KOH Lösung gelöst. Zum Schluss wurde das Monopropylengykol zugegeben.

### Resultate :

| Probelösung | |
|---|---|
| | Kristallisation bei -20°C |
| | Unter Impfen mit 100 mg |
| | OPP als Impfkristalle |
| 50 Gew.% Phenol (105 Mol% K-neutr.) mit 5 % Monopropylenglykol | keine Kristallisation |

### Beispiel 15

Probelösung 50 Gew.%ig + 5 % Monopropylenglykol :

| | |
|---|---|
| 500,0 g | o-Kresol |
| 272,2 g | KOH |
| 50,0 g | Monopropylenglykol, |
| 177,8 g | Wasser |

Das Wasser wurde jeweils vorgelegt, die KOH zugegeben und innert 5 Min. unter Rühren auf dem Magnetrührer gelöst. Anschliessend wurde das o-Kresol zugegeben und bei 50°C unter Rühren auf dem Magnetrührer in der KOH Lösung gelöst. Zum Schluss wurde das Monopropylengykol zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Kresol (105 Mol-% K-neutr.) mit 5 % Monopropylenglykol | keine Kristallisation |

### Beispiel 16

Probelösung 50 Gew.%ig + 5 % Monopropylenglykol + 0,2 Oxidationsinhibitor:

| | |
|---|---|
| 500,0 g | o-Phenylphenol |
| 173,0 g | KOH |
| 50,0 g | Monopropylenglykol |
| 2,0 g | 2-Phosphono-1,2,4-butantricarbonsäure |
| 275,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das Monopropylengykol und der Oxidationsstabilisator zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (OPP) (105 Mol-% K-neutr.) mit 5 % Monopropylenglykol | keine Kristallisation |

Eine 50 Gew.%ige Lösung von o-Phenylphenol, neutralisiert mit 1,05 Mol KOH pro Mol o-Phenylphenol, entsprechend 66.5 Gew.% Kalium-o-phenylphenolat, in destilliertem Wasser kristallisiert auch unter Zugabe von OPP als Impfkristalle bei - 20°C nicht aus, wenn der erfindungsgemässe Kristallisationshemmer in Form von Monopropylenglykol zugegeben wird. Der Oxidationsstabilisator beeinflusst die Eigenschaftenen der Formulierung nicht negativ.

### Beispiel 17

Probelösung 50 Gew.%ig + 5 % Monopropylenglykol + 0,05 % Komplexbildner:

| | |
|---|---|
| 500,0 g | o-Phenylphenol |
| 173,0 g | KOH |
| 50,0 g | Monopropylenglykol |
| 0,5 g | Ethylendiamintetraessigsäure - Dinatrium-Salz (EDTA) |
| 276,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das Monopropylengykol und der Komplexbildner zugegeben.

### Resultate :

| Probelösung | |
|---|---|
| | Kristallisation bei -20°C |
| | Unter Impfen mit 100 mg |
| | OPP als Impfkristalle |
| 50 Gew.% o-Phenylphenol (OPP) (105 Mol-% K-neutr.) mit 5 % Monopropylenglykol | keine Kristallisation |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe von OPP als Impfkristalle bei - 20°C nicht aus, wenn der erfindungsgemässe Kristallisationshemmer in Form von Monopropylenglykol zugegeben wird. Der Komplexbildner beeinflusst die Eigenschaften der Formulierung nicht negativ.

### Beispiel 18

Probelösung 50 Gew.%ig + 5 % Monopropylenglykol + 1 % die biozide Wirkung unterstützende Substanz

| | |
|---|---|
| 500,0 g | o-Phenylphenol |
| 173,0 g | KOH |
| 50,0 g | Monopropylenglykol |
| 10,0 g | Pepton |
| 267,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol zugegeben und bei 50°C unter Rühren auf dem Magnetrührer in der KOH Lösung gelöst. Zum Schluss wurde das Monopropylengykol und die die biozide Wirkung unterstützende Substanz zugegeben.

### Resultate :

| Probelösung | |
|---|---|
| | Kristallisation bei -15°C |
| | Unter Impfen mit 100 mg |
| | OPP als Impfkristalle |
| 50 Gew.% o-Phenylphenol (OPP) (105 Mol-% K-neutr.) mit 5 % Monopropylenglykol | keine Kristallisation |
| und 1 % der die biozide Wirkung unterstützende Substanz | |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe von OPP als Impfkristalle bei -15°C nicht aus, wenn der erfindungsgemässe Kristallisationshemmer in Form von Monopropylenglykol zugegeben wird. Das Pepton beeinflusst die Eigenschaften der Formulierung nicht negativ.

### Beispiel 19

Probelösung 50 Gew.%ig + 7 % Monopropylenglykol + 3 % weitere, biozid wirkende. Substanz

| | |
|---|---|
| 500,0 g | o-Phenylphenol |
| 173,0 g | KOH |
| 70,0 g | Monopropylenglykol |
| 50,0 g | N-Tallow-1,3-diaminopropan |
| 207,0 g | Wasser |

Das Wasser wurde jeweils vorgelegt, die KOH zugegeben und innert 5 Min. unter Rühren auf dem Magnetrührer gelöst. Anschliessend wurde das o-phenylphenol zugegeben und bei 50°C unter Rühren auf dem Magnetrührer in der KOH Lösung gelöst. Zum Schluss wurden das Monopropylengykol und die weiteren biozid wirkende Substanz zugegeben.

### Resultate :

| Probelösung | |
|---|---|
| | Kristallisation bei -15°C |
| | Unter Impfen mit 100 mg |
| | OPP als Impfkristalle |
| 50 Gew.% o-Phenylphenol (OPP) (105 Mol-% K-neutr.) mit 7 % Monopropylenglykol | keine Kristallisation |
| und 5 % der weiteren biozid wirkenden Substanz | |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destilliertem Wasser kristallisiert auch unter Zugabe von OPP als Impfkriställe bei -15°C nicht aus, wenn der erfindungsgemässe Kristallisationshemmer in Form von Monopropylenglykol zugegeben wird. Das weitere Biozid beeinflusst die Eigenschaften der Formulierung nicht negativ.

### Beispiel 20

Probelösung 50 Gew.%ig + 3 % Monopropylenglykol +

| | |
|---|---|
| 500,0 g | Trichlorphenol |
| 150,0 g | KOH |
| 30,0 g | Monopropylenglykol |
| 320,0 g | Wasser |

Das Wasser wurde jeweils vorgelegt, die KOH zugegeben und innert 5 Min. unter Rühren auf dem Magnetrührer gelöst. Anschliessend wurde das Trichlorphenol zugegeben und bei 50°C unter Rühren auf dem Magnetrührer in der KOH Lösung gelöst. Zum Schluss wurde das Monopropylengykol zugegeben.

### Resultate :

| Probelösung | |
|---|---|
| | Kristallisation bei -15°C |
| | Unter Impfen mit 100 mg |
| | OPP als Impfkristalle |
| 50 Gew.% Trichlorphenol (105 Mol-% K-neutr.) mit 3 % Monopropylenglykol | keine Kristallisation |

Eine 50 Gew.%ige Lösung von Trichlorphenol, neutralisiert mit 1,05 Mol KOH pro Mol Trichlorphenol, entsprechend 60 Gew.% Kalium-Trichlorphenolat, in destilliertem Wasser kristallisiert auch unter Zugabe von OPP als Impfkristalle bei -15°C nicht aus, wenn der erfindungsgemässe Kristallisationshemmer in Form von Monopropylenglykol zugegeben wird.

### Beispiel 21

Probelösung 50 Gew.%ig + 5 % Monopropylenglykol + 5 % weitere, biozid wirkende Substanz

| | |
|---|---|
| 500,0 g | o-Phenylphenol |
| 173,0 g | KOH |
| 50,0 g | Monopropylenglykol |
| 50,0 g | Natriumsalizylat |
| 227,0 g | Wasser |

Das Wasser wurde jeweils vorgelegt, die KOH zugegeben und innert 5 Min. unter Rühren auf dem Magnetrührer gelöst. Anschliessend wurde das o-Phenylphenol und die weitere, biozid wirkende Substanz zugegeben und bei 50°C unter Rühren auf dem Magnetrührer in der KOH Lösung gelöst. Zum Schluss wurde das Monopropylengykol zugegeben.

### Resultate :

| Probelösung | |
|---|---|
| | Kristallisation bei -15°C |
| | Unter Impfen mit 100 mg |
| | OPP als Impfkristalle |
| 50 Gew.% o-Phenylphenol (OPP) (105 Mol-% K-neutr.) mit 5 % Monopropylenglykol | keine Kristallisation |
| und 5 % der weiteren biozid wirkenden Substanz | |

Eine 50 Gew.%ige Lösung von o-Phenylphenol, neutralisiert mit 1,05 Mol KOH pro Mol o-Phenylphenol, entsprechend 66,5 Gew.% Kalium-o-phenylphenolat, in destillierten Wasser kristallisiert auch unter Zugabe von OPP als Impfkristalle bei -15°C nicht aus wenn der erfindungsgemässe Kristallisationshemmer in Form von Monopropylenglyko zugegeben wird. Das weitere Biozid beeinflusst die Eigenschaften der Formulierung nicht negativ.

### Beispiel 22

Probelösung 50 Gew.%ig + 5 % Monopropylenglykol

| | |
|---|---|
| 500,0 g | o-Kresol |
| 174,9 g | KOH |
| 50,0 g | Monopropylenglykol |
| 275,1 g | Wasser |

Das Wasser wurde jeweils vorgelegt, die KOH zugegeben und innert 5 Min. unte Rühren auf dem Magnetrührer gelöst. Anschliessend wurde das o-Kresol zugegeber und bei 50°C unter Rühren auf dem Magnetrührer in der KOH Lösung gelöst. Zun Schluss wurde das Monopropylengykol zugegeben.

### Resultate :

| Probelösung | |
|---|---|
| | Kristallisation bei -15°C |
| | Unter Impfen mit 100 mg |
| | o-Kresol als Impfkristalle |
| 50 Gew.% o-Kresol (105 Mol-% K-neutr.) mit 5 % Monopropylenglykol | keine Kristallisation |

Eine 50 Gew.%ige Lösung von o-Kresol, neutralisiert mit 1,05 Mol KOH pro Mol o-Phenylphenol, entsprechend 61,6 Gew.% o-Kresol-Kaliumsalz, in destilliertem Wasse kristallisiert auch unter Zugabe von o-Kresol als Impfkristalle bei - 15°C nicht aus wenn der erfindungsgemässe Kristallisationshemmer in Form von Monopropylenglyko zugegeben wird.

## Patentansprüche

1. Wässrige, Phenolate enthaltende flüssige Formulierung mit einem Erstarrungspunkt von kleiner oder gleich -10°C,
**dadurch gekennzeichnet,**
**daß** sie die folgenden Bestandteile enthält :
a) 50-80 Gew.-% eines oder mehrerer Phenolate;
b) 0,1-10 Gew.-% mindestens eines Kristallisationshemmers ausgewählt aus einer oder mehreren aliphatischen Glykolverbindungen wie Ethylenglycol, Monopropylenglykol und/oder Diethylenglycol, und/oder Ben-zylalkohol, 2-Phenylethan-1-ol, 3-Phenylpropan-1-ol und/oder 1-Phenylpropan-2-ol ; und die Differenz zu jeweils 100 Gew.-% der Alkaliüberschuss von 0.03-0.15 Mol/Mol und Wasser ist.

2. Formulierung nach Anspruch 1,
**dadurch gekennzeichnet,**
**daß** sie als Phenolate Salze des Phenols und/oder einfach oder mehrfach substituierte Phenolate mit aliphatischen und/oder aromatischen Substituenten enthält.

3. Formulierung nach Anspruch 1 oder 2,
**dadurch gekennzeichnet,**
**daß** sie als Phenolate o-Phenylphenolate und/oder halogenierte Phenolate und/oder Salze der Kresole und/oder Salze halogenierter Kresole und/oder Salze der Resorcine enthält.

4. Formulierung nach Anspruch 1 zu 3,
**dadurch gekennzeichnet,**
**daß** sie die Phenolate in einer Menge von 50-75 Gew.-%, bevorzugt 55-70 Gew.-% und weiterhin bevorzugt 60-70 oder 62-67 Gew.-% enthält.

5. Formulierung nach einem oder mehreren der vorhergehenden Ansprüche,
**dadurch gekennzeichnet,**
**daß** sie die Kristallisationshemmer in einer Menge von 0,5-5,0 Gew.-%, bevorzugt 1-3 Gew.-% enthält.

6. Formulierung nach einem oder mehreren der vorhergehenden Ansprüche,
**dadurch gekennzeichnet,**
**daß** sie als zusätzliche mikrobiozide Mittel metallorganische Verbindungen und/oder quaternäre Ammoniumverbindungen und/oder als mikrobiozide Mittel unterstützende Substanzen Komplexbildner und/oder Antioxidantien (Oxidationsstabilisatoren) enthält.

7. Formulierung nach einem oder mehreren der vorhergehenden Ansprüche,
**dadurch gekennzeichnet,**
**daß** als mikrobiozide Mittel, Dicocomethylbenzylammonium chlorid und/oder Tributylzinnbenzoat und/oder N-Tallow-1,3-diaminopropan enthalten sind.

8. Formulierung nach einem oder mehreren der vorhergehenden Ansprüche,
**dadurch gekennzeichnet,**
**daß** als Komplexbildner NTA und/oder EDTA und/oder DTPA und/oder als Antioxidans 2-Phosphono-1,2,4,-butantricarbonsäure, bevorzugt jeweils in Mengen von 0,05 - 1 Gew.-% bezogen auf die Formulierung, enthält.

9. Formulierung nach einem oder mehreren der vorhergehenden Ansprüche,
**dadurch gekennzeichnet,**
**daß** die Formulierung
a) 50-80 Gew.-% zumindest eines Phenolats und
b) 20-50 Gew.-% eines Lösungsmittelsystems enthält, welches zu 90-99 Gew.-% aus Wasser und zu 0,1-10,0 Gew.-% aus zumindest einem Kristallisationshemmer besteht, wobei ein Anteil von 1,0-4,9 Gew.-% dieser Formulierung durch weitere mikrobiozide Mittel und/oder diese Mittel unterstützende Bestandteile ersetzt sein kann.

10. Formulierung nach einem oder mehreren der vorhergehenden Ansprüche,
**dadurch gekennzeichnet,**
**daß** die Phenolate als Kalium-Salz oder Kalium- und Natrium- und/oder Kaliumund Lithium-Salz vorliegen.

11. Formulierung nach einem oder mehreren der vorhergehenden Ansprüche,
**dadurch gekennzeichnet,**
**daß** pro Mol Phenolat zur Neutralisation 1,03-1,15, bevorzugt 1,05-1,10 Mol an Alkalihydroxyden eingesetzt wurden.

12. Formulierung nach einem oder mehreren der vorhergehenden Ansprüche,
**dadurch gekennzeichnet,**
**daß** die Kristallisationshemmer in einer Menge von 1-3 Gew.-% in einer 60-70 Gew.-% Phenolat enthaltenden Formulierung vorliegen.

13. Wässrige Suspension oder Dispersion von Mineralien und/oder Füllstoffen und/oder Pigmenten und/oder natürlichen oder synthetischen organischen Bindemitteln und/oder Kühlschmierstoffen, enthaltend eine Formulierung nach einem oder mehreren der vorhergehenden Ansprüche.

14. Suspension oder Dispersion nach Anspruch 13,
**dadurch gekennzeichnet,**
**daß** die Formulierung in einer Menge von 100g/Tonne - 2500 g/Tonne Suspension oder Dispersion vorliegt.

15. Verwendung einer Formulierung nach einem oder mehreren der vorhergehenden Ansprüche als Konservierungsmittel in einer wässrigen Suspension oder Dispersion von Mineralien und/oder Füllstoffen und/oder Pigmenten und/oder natürlichen oder synthetischen organischen Bindemitteln und/oder Kühlschmierstoffen.

16. Verwendung nach Anspruch 15,
**dadurch gekennzeichnet, daß**
das Konservierungsmittel in der Metall bearbeitenden Industrie, bei der Papierherstellung, Papierbeschichtung, in wässrigen Lacken und in Farben eingesetzt wird.

17. Verwendung einer Formulierung nach einem oder mehreren der Ansprüche 15 und 16 als Konservierungsmittel,
**dadurch gekennzeichnet,**
**daß** sie als Konservierungs- und/oder Beizmittel in der Holz verarbeitenden Industrie und/oder im Forstbereich eingesetzt wird.

18. Verfahren zur Herstellung einer Phenolate enthaltenden Formulierung nach einem oder mehreren der vorhergehenden Ansprüche,
**dadurch gekennzeichnet,**
**daß** Wasser und das Neutralisationsmittel vorgelegt und die Phenolverbindungen darin gelöst werden und anschliessend der Kristallisationshemmer zugegeben wird.

19. Verfahren nach Anspruch 18,
**dadurch gekennzeichnet, daß**
der Kristallisationshemmer zusammen mit dem Wasser und dem Neutralisationsmittel vorgelegt werden anschliessend die Phenolverbindung darin gelöst werden.

20. Verfahren nach Anspruch 18 oder 19,
**dadurch gekennzeichnet, daß**
die Temperatur beim Lösungsvorgang 5-80°C, bevorzugt 40-60 °C beträgt.

## Claims

1. Aqueous, phenolates-containing liquid formulation having a solidification point of less than or equal to -10°C, **characterized in that** said formulation contains the following components:
a) 50 - 80% by weight of one or more phenolates;
b) 0.1 - 10% by weight of at least one crystallization inhibitor selected from one or more aliphatic glycol compound(s), such as ethylene glycol, monopropylene glycol and/or diethylene glycol, and/or benzyl alcohol, 2-phenylethane-1-ol, 3-phenylpropane-1-ol and/or 1-phenylpropane-2-ol; and the balance with respect to 100% by weight is comprised by alkali in an excess of 0.03-0.15 mol/mol and water.

2. Formulation according to claim 1, **characterized in that** it contains as said phenolates salts of phenol and/or phenolates having one or more aliphatic and/or aromatic substituents.

3. Formulation according to claim 1 or 2, **characterized in that** it contains as said phenolates o-phenylphenolates and/or halogenated phenolates and/or cresol salts and/or salts of halogenated cresols and/or resorcinol salts.

4. Formulation according to any one of claims 1 to 3, **characterized in that** it contains the phenolates in an amount of 50 - 75% by weight, preferably 55 - 70% by weight, and further preferred 60 - 70 or 62 - 67% by weight.

5. Formulation according to one or more of the preceding claims, **characterized in that** it contains the crystallization inhibitors in an amount of 0.5 - 5% by weight, preferably 1 - 3% by weight.

6. Formulation according to one or more of the preceding claims, **characterized in that** it contains organometal compounds and/or quaternary ammonium compounds as the additional microbicidal agents, and/or chelating agents and/or antioxidants (oxidation stabilizers) as the substances promoting the microbicidal agent(s).

7. Formulation according to one or more of the preceding claims, **characterized in that** dicocomethylbenzylammonium chloride and/or tributyl tin benzoate and/or N-tallow-1,3-diaminopropane are comprised as the microbicidal agents.

8. Formulation according to one or more of the preceding claims, **characterized in that** it contains NTA and/or EDTA and/or DTPA as the chelating agent, and/or 2-phosphono-1,2,4-butanetricarboxylic acid as the antioxidant, each preferably in an amount of 0.05 - 1% by weigth based on the formulation.

9. Formulation according to one or more of the preceding claims, **characterized in that** said formulation contains:
a) 50 - 80% by weight of at least one phenolate; and
b) 20 - 50% by weight of a solvent system comprising 90 - 99% by weight water and 0.1 - 10,0% by weight of at least one crystallization inhibitor; wherein a proportion of 1.0 - 4.9% by weight said formulation may be replaced by additional microbicidal agents and/or components promoting these agents.

10. Formulation according to one or more of the preceding claims, **characterized in that** said phenolates are present in the form of the potassium salt or the potassium and sodium salts and/or the potassium and lithium salts.

11. Formulation according to one or more of the preceding claims, **characterized in that** 1.03 - 1.15, preferably 1,05 - 1.10 mols of alkali hydroxides per mol of phenolate have been used for neutralization.

12. Formulation according to one or more of the preceding claims, **characterized in that** the crystallization inhibitors are present in an amount of 1 - 3% by weight in a formulation containing 60 - 70% by weight of phenolate.

13. Aqueous suspension or dispersion of minerals and/or fillers and/or pigments and/or natural or synthetic organic binders and/or cooling lubricants containing a formulation according to one or more of the preceding claims.

14. Suspension or dispersion according to claim 13, **characterized in that** the formulation is present in an amount of 100 g/tonne to 2500 g/tonne of the suspension or dispersion.

15. Use of a formulation according to one or more of the preceding claims as a preservative agent in an aqueous suspension or dispersion of minerals and/or fillers and/or pigments and/or natural or synthetic organic binders and/or cooling lubricants.

16. Use according to claim 15, **characterized in that** said preservative is employed in the metal industry, in papermaking, paper coating, in aqueous lacquers and in paints.

17. Use of a formulation according to one or more of claims 15 and 16 as a preservative, **characterized in that** said formulation is employed as a preservative and/or mordant in the wood industry and/or in forestry.

18. Method for the preparation of a phenolates-containing formulation according to one or more of the preceding claims, **characterized in that** water and a neutralizing agent are placed in a vessel, and dissolving the phenol compounds therein, and afterwards adding the crystallization inhibitor.

19. Method according to claim 18, **characterized in that** the crystallization inhibitor is placed together with the water and the neutralizing agent in a vessel, and afterwards the phenol compound are dissolved therein.

20. Method according to claim 18 or 19, **characterized in that** the temperature during dissolving is 5 - 80°C, preferably 40 - 60°C.

## Revendications

1. Formulation liquide aqueuse contenant des phénolates et ayant un point de solidification inférieur ou égal à -10°C, **caractérisée en ce qu'**elle contient les constituants suivants :
a) 5 à 80% en poids d'un ou de plusieurs phénolates ;
b) 0,1 à 10% en poids d'au moins un inhibiteur de cristallisation choisi parmi un ou plusieurs composés aliphatiques de type glycol tels que l'éthylène glycol, le monopropylène glycol et/ou le diéthylène glycol et/ou parmi l'alcool benzylique, le 2-phényléthan-1-ol, le 3-phénylpropan-1-ol et/ou le 1-phénylpropan-2-ol ; le complément permettant d'atteindre 100% en poids correspondant à l'excédent en métaux alcalins de 0,03 à 0,15 mol/mol et à l'eau.

2. Formulation selon la revendication 1, **caractérisée en ce qu'**elle contient, en tant que phénolates, des sels de phénol et/ou des phénolates pourvus d'un ou de plusieurs substituants aliphatiques et/ou aromatiques.

3. Formulation selon les revendications 1 ou 2, **caractérisée en ce qu'**elle contient, en tant que phénolates, des o-phénylphénolates et/ou des phénolates halogénés et/ou des sels de crésols et/ou des sels de crésols halogénés et/ou des sels de résorcines.

4. Formulation selon les revendications 1 à 3, **caractérisée en ce qu'**elle contient lesdits phénolates dans une quantité comprise entre 50 et 75% en poids, de préférence entre 55 et 70% en poids, et encore plus préférentiellement entre 60 et 70 ou entre 62 et 67% en poids.

5. Formulation selon l'une ou plusieurs des revendications précédentes, **caractérisée en ce qu'**elle contient lesdits inhibiteurs de cristallisation dans une quantité comprise entre 0,5 et 5,0% en poids, de préférence entre 1 et 3% en poids.

6. Formulation selon l'une ou plusieurs des revendications précédentes, **caractérisée en ce qu'**elle contient en outre, en tant qu'agents microbicides, des composés organométalliques et/ou des composés d'ammonium quaternaires et/ou, en tant que substances renforçant l'effet des agents microbicides, des agents complexants et/ou des antioxydants (agents de stabilisation vis-à-vis de l'oxydation).

7. Formulation selon l'une ou plusieurs des revendications précédentes, **caractérisée en ce qu'**elle contient, en tant qu'agents microbicides, du chlorure de dicocométhylbenzylammonium et/ou du benzoate de tributylétain et/ou du N-tallow-1,3-diaminopropane.

8. Formulation selon l'une ou plusieurs des revendications précédentes, **caractérisée en ce qu'**elle contient, en tant agents complexants, du NTA et/ou de l'EDTA et du DTPA et/ou, en tant qu'antioxydant, de l'acide 2-phosphono-1,2,4-butancarboxylique, les quantités en étant chacune préférentiellement comprise 0,05 et 1% en poids par rapport à la formulation.

9. Formulation selon l'une ou plusieurs des revendications précédentes, **caractérisée en ce que** ladite formulation contient:
a) 50 à 80% en poids d'au moins un phénolate et
b) 20 à 50% en poids d'un système de solvants constitué à 90 à 99% en poids d'eau et à 0,1 à 10,0% en poids d'au moins un inhibiteur de cristallisation, une proportion comprise entre 1,0 et 4,9% en poids de cette formulation pouvant être remplacée par d'autres agents microbicides et/ou par des constituants renforçant l'effet desdits agents.

10. Formulation selon l'une ou plusieurs des revendications précédentes, **caractérisée en ce que** lesdites phénolates se présentant sous forme d'un sel de potassium ou d'un sel de potassium et de sodium et/ou de potassium et de lithium.

11. Formulation selon l'une ou plusieurs des revendications précédentes, **caractérisée en ce que** l'on met en oeuvre, par mol de phénolate, 1,03 à 1,15, de préférence 1,05 à 1,10 mol d'hydroxydes de métaux alcalins, pour ainsi effectuer la neutralisation.

12. Formulation selon l'une ou plusieurs des revendications précédentes, **caractérisée en ce que**, dans une formulation contenant 60 à 70% en poids de phénolate, lesdits inhibiteurs de cristallisation sont présents dans une quantité comprise entre 1 et 3% en poids.

13. Suspension ou dispersion aqueuse de minéraux et/ou de charges et/ou de pigments et/ou de liants organiques naturels ou synthétiques et/ou de réfrigérants lubrifiants, contenant une formulation selon l'une ou plusieurs des revendications précédentes.

14. Suspension ou dispersion selon la revendication 13, **caractérisée en ce que** ladite formulation est présente dans une quantité comprise entre 100 g/tonne et 2 500 g/tonne de suspension ou dispersion.

15. Utilisation d'une formulation selon l'une ou plusieurs des revendications précédentes en tant que conservateur dans une suspension ou dispersion aqueuse de minéraux et/ou de charges et/ou de pigments et/ou de liants organiques naturels ou synthétiques et/ou de réfrigérants lubrifiants.

16. Utilisation selon la revendication 15, **caractérisée en ce que** ledit conservateur est mis en oeuvre dans l'industrie de transformation des métaux, dans la fabrication de papier, le couchage de papier, dans des vernis à base d'eau et dans des peintures.

17. Utilisation d'une formulation selon une ou plusieurs des revendications 15 et 16 en tant que conservateur, **caractérisée en ce qu'**elle est mise en oeuvre en tant que conservateur et/ou décapant dans l'industrie de transformation du bois et/ou dans le domaine forestier.

18. Procédé de préparation d'une formulation contenant des phénolates selon l'une ou plusieurs des revendications précédentes, **caractérisé en ce que** l'on dissout les composés phénoliques en les intégrant à un mélange constitué d'eau et de l'agent de neutralisation, pour ensuite y ajouter l'inhibiteur de cristallisation.

19. Procédé selon la revendication 18, **caractérisé en ce que** l'on dissous le composé phénolique en l'intégrant à un mélange constitué d'eau, de l'agent de neutralisation et de l'inhibiteur de cristallisation.

20. Procédé selon les revendications 18 ou 19, **caractérisé en ce que** le processus de dissolution est réalisé à une température comprise entre 5 et 80°C, de préférence entre 40 et 60°C.
